# EUROPEAN PATENT SPECIFICATION

(11) **EP 1 893 082 B1**
(45) Date of publication and mention of the grant of the patent: **22.09.2010**
(21) Application number: 05769817.7
(22) Date of filing: 14.06.2005
(51) Int. Cl.: A61B 5/021, G06F 19/00

(54) **A BLOOD PRESSURE MEASURING DEVICE AND A METHOD FOR OPERATING A BLOOD PRESSURE MEASURING DEVICE**
BLUTRUCKMESSGERÄT UND VERFAHREN ZUR BEDIENUNG EINES BLUTDRUCKMESSGERÄTS
DISPOSITIF DE MESURE DE LA TENSION ARTERIELLE ET PROCEDE POUR LE FONCTIONNEMENT D'UN DISPOSITIF DE MESURE DE LA TENSION ARTERIELLE

(43) Date of publication of application: 05.03.2008
(73) Proprietor: Microlife Intellectual Property GmbH, 9443 Widnau (CH)
(72) Inventor: LIN, Kin-Yuan, Taipei (TW)
(74) Representative: Hepp, Dieter
(86) International application number: PCT/EP2005/052739
(87) International publication number: WO 2006/133735

(56) References cited:
- EP-A- 0 960 598
- EP-A- 1 224 903
- WO-A-02/053024
- US-A- 4 944 305
- US-A- 5 967 975
- US-B1- 6 450 955
- US-B2- 6 699 195
- DATABASE BIOSIS [Online] BIOSCIENCES INFORMATION SERVICE, PHILADELPHIA, PA, US; June 1998 (1998-06), STERGIOU GEORGE S ET AL: "Self-monitoring of blood pressure at home: How many measurements are needed?" XP002369023 Database accession no. PREV199800342943 cited in the application & STERGIOU GEORGE S ET AL: "Self-monitoring of blood pressure at home: How many measurements are needed?" JOURNAL OF HYPERTENSION, vol. 16, no. 6, June 1998 (1998-06), pages 725-731, ISSN: 0263-6352

## Description

The invention relates to a blood pressure measuring device and to a method for operating a blood pressure measuring device.

Blood pressure measuring devices for self-monitoring of blood pressure at home are widely used today. In view of diagnostics of hypertension, it is much easier for a patient to verify the blood pressure at home instead of going to a hospital or to a doctor. Home measurements also allow to acquire a plurality of flood pressure measurement data.

In EP 1224903 A1 a blood pressure measuring device is disclosed, which has a voice generator to remind a patient to take a certain medicine and/or to perform blood pressure measurement according to a schedule.

WO 02/053024 A2 discloses a system for measuring blood pressure being able to remind the patient to follow a predetermined measurement schedule stored in the device.

US 6699195 B2 describes a blood pressure measurement device including an alarm for reminding a patient to perform a blood pressure measurement according to a measurement schedule which is adapted to the habits of the user.

US 5967975 A shows a method for centrally monitoring the times of performance of home measurements of health parameters, e.g. blood pressure. In case the measurement is not performed on time according to prescribed times intervals the user can be reminded by a central system, therefore assuring that home measurements are performed before much time elapses.

US 6450955 B1 discloses a system which is able to provide a reminder or a series of reminders to the user that measurements need to be taken.

With a recommended validated blood pressure measuring device, the self-monitoring of blood pressure at home can be more effective and convenient than conventional measurements. Home blood pressure measurements can prevent from common known "white-coat hypertension" or "effect" which may lead to overdose or inappropriate medication. The recent clinical reports also show that home BP measurements can have high predictive power and reliability for the hypertension and cardiovascular disease.

It is known that most people lack for the right knowledge of the procedures for self-monitoring of blood pressure.

Thus the measurement values of outpatients measurements are, however, sometimes not very reliable. In particular, it is known that the measurement made on the first day of a series of subsequent measurements may falsify the complete measurement sequence. In addition, the specific time when measurements are made is of certain relevance. Furthermore, if a patient forgets to make regular measurements, the results may not be reliable enough for making diagnostics.

It is therefore an object of the present invention to provide a blood pressure measuring device and a method for operating such a device which allows patients to self-monitor their blood pressure in view of a subsequent diagnostics to be made by a doctor. According to the present invention, these and other objects are solved according to the independent patent claims.

The present invention provides a blood pressure measuring device according to claim 1.

The blood pressure monitoring device according to the invention comprises an alarm generator. The alarm generator is designed such as to remind the patient to carry out a blood pressure measurement or to automatically witch the device on according to a predetermined measurement schedule. The measurement schedule is typically based on clinically tested protocols which have turned out to lead to reliable measurements. By including such an alarm generator, it is made sure that the patient does not forget to make regular measurements. In view of diagnostics of hypertension, this invention can guide users how to do the recommended validated procedures of self-monitoring of blood pressure at home, which will enhance the reading's accuracy and patient's compliance.

Preferably, the measurement schedule is stored in a memory within the device. It is possible to have a predefined measurement schedule stored in a read only memory. Alternatively, it is also possible to store a specific measurement schedule for an individual patient. Such an individual measurement schedule can be defined by a doctor.

The device further may comprise a memory for storing the results of a plurality of measurements made according to the measurement schedule.

In particular, it is possible to provide the device with a calculating means for forming averages of the results from at least two measurements. In particular, the calculating means can be used for forming daily averages based on at least two measurements on one day. They may also be used for forming averages of a plurality of such daily measurement results, in particular of such daily averages.

The device further may be provided with a means for displaying the averages. Such means allow the user to verify the current average from time to time, e. g. in the evening of each day.

In particular, it is known that the measurement made on the first day of a series of subsequent measurement may falsify the complete measurement sequence. By using the accuracy reading, a medical doctor can be more distinct to diagnose patient's true level of hypertension. According to a preferred embodiment of the invention, the calculation means for forming the averages are designed such as to disregard measurements which have been made on the first day of a series of subsequent measurement days.

The measurement schedule may generate an alarm daily, in particular in the morning and in the evening. Such alarm can be repeated for a predetermined number of days, e.g. for at least three days, in particular for seven subsequent days. In addition to generating an alarm, the device is designed in such a way that measurements can only be made in a certain time window, e. g. 3 hours around the alarm time. The patient thus can decide to make later or earlier measurement, but still within a time window which leads to reliable results.

The device according to the present invention is particularly suitable for carrying out measurements according to verified blood pressure measurement recommendations. Such recommendations are e. g. known from article "Self-monitoring of blood pressure at home: how many measurements are needed?" (George S. Stergiou, in Journal of Hypertension 1998, Vol. 16, no. 6, page 725) from the "European Society of Hypertension recommendations for conventional, ambulatory and home blood pressure measurement" (Eoin O'Brien et al, published in Journal of Hypertension, 2003, Vol. 21, no. 5, page 821).

According to a further preferred embodiment of the invention, the device may be operated in different operating modes. The operation mode as described herein above is a so-called diagnostic mode. It is primarily used in advance of diagnostics by a doctor. The device also may be operated in a regular measurement mode and/or in a so-called therapeutic mode. In the regular mode, the device is operating in a manner comparable to a current state of the art blood pressure monitoring devices. In the therapeutic mode, the device may operate in a similar way as in the diagnostic mode. The number of consecutive measurement days however may be larger, typically extend to more than two weeks. The device is operable in the therapeutic decision mode, which can monitor the blood pressure for assessing the effect of antihypertensive drug treatment. In this mode, the alarm generator works according to a predetermined daily measurement. The alarm generator is designed in such a way as to remind the patient to carry out a blood pressure measurement and the following drug intake

In particular, the device may be provided with a manual switch for switching between these operating modes. The switch may be mechanical. It may also be electronic by means of a touch screen or by means of set-up buttons in combination with information displayed on a display screen.

According to another embodiment of the invention, the device may furthermore comprise means for storing the occurrence of an irregular heartbeat and means for calculating the occurrence percentage of irregular heartbeats measurements within a predetermined period of time of measurement. The device is furthermore provided with means for displaying such a occurrence percentage. It is already known to measure irregular heartbeats or arrhythmia automatically and in parallel during blood pressure measurements. Such a device and method is e. g. disclosed in EP 960 598. According to the present invention, irregular heartbeat detection may e. g. be made in accordance with this disclosure. Other known means for detection may also be used. This aspect of the invention allows the user to have more reliable information relating to irregular heartbeats than just information relating to a single occurrence of an irregular heartbeat. It's proven that there is a high correlation of results between self-monitoring and ambulatory blood pressure measurements. It is also known that doctors have a difficulty in treatment for many complicated cases of hypertension. In view of medication treatment of hypertension, by using similar procedures, this invention can also monitor the effect of treated medication at home in a convenient and economical way. The indication for rising or descending occurrence probability of irregular heartbeat is designed such as to provide the doctor with a reference Index for assessing the types and doses of treated antihypertensive drug.

In particular, the device may furthermore be provided with means for determining whether there is an increase or a decrease of this occurrence percentage in the course of time measurement. While such an irregular heartbeat monitoring device in a blood pressure monitor is particularly advantageous in context with the device according to a predetermined measurement schedule, the irregular heartbeat indication can also be useful in context with other blood pressure measuring devices.

According to the invention, there is provided a method for operating a blood pressure measuring device as defined in claim 12. In particular, the method is applicable to devices as described herein above. In a first step, there is provided a predetermined measurement schedule in the device. This may be a preset schedule, e. g. set by the manufacturer of the device. It may also be a schedule which is provided on a patient individual basis in a memory within the device. When certain criteria of the measurement schedule are fulfilled, an alarm signal is generated or the device is automatically turned on. The alarm signal reminds the patient to carry out a measurement.

The alarm typically is generated daily, in the morning and in the evening. In particular, it can be generated for a plurality of successive days, in particular seven days for diagnostic purposes. It is also possible to create alarms for a larger number of days, in particular for therapeutic purposes. Operation of the device during times outside a time window around the alarm time is blocked in the diagnostic mode.

According to an embodiment of the invention it is also possible to form averages of a plurality of measured values. In particular, daily averages and averages of a plurality of subsequent daily measurements or daily averages may be formed.

For determination of averages, it is preferred to disregard the measurement values which have been made on the first day of the measurement schedule.

The invention.will now be explained in more details in the following embodiments with reference to the accompanying drawings, which show:
- Fig. 1: a schematic representation of a blood pressure measur- ing device according to the invention
- Fig. 2: a schematic representation of several modules of a de- vice according to the invention
- Fig. 3: an enlarged view of a display and user interface of a device according to the present invention
- Fig. 4 and 5: Flow charts of the operation of a device according to the invention in two different operating modes and
- Fig 6 and 7: Flow charts of the operation of a device according to the invention with irregular heart beat detection.

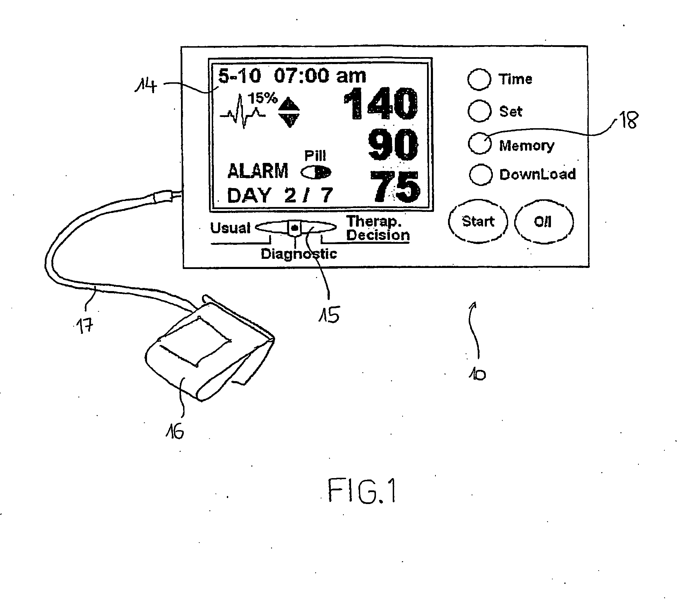

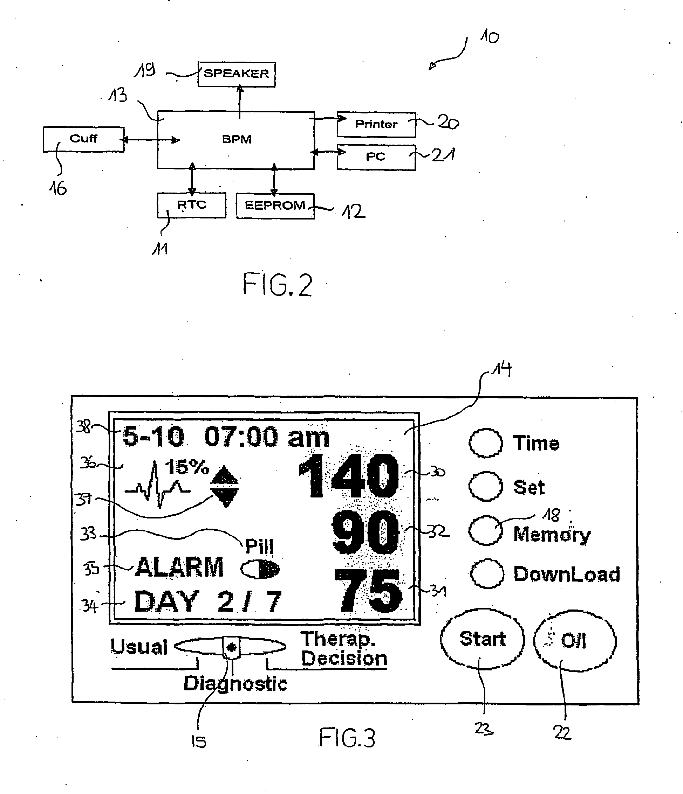

Fig. 1 shows a blood pressure measuring device 10. The blood pressure measuring device 10 comprises a main unit in a housing. A cuff 16 is attached to the housing by means of a tube 17. The cuff is adapted to be placed around an upper arm of the user. The blood pressure measuring unit works in a manner known in the art, in particular by inflating the cuff and slowly decreasing the pressure in the cuff while measuring the pressure within cuff. Based on the pressure signals and the blood pressure is determined in the oscillometric method. The device is provided with a display 14 for proving a plurality of measurement data and user instructions. Furthermore, the device is provided with operation buttons such as a memory button 18 for displaying stored results (see also Fig. 3).

According to Fig. 2, the blood pressure monitor 10 comprises a calculating unit 13 which controls operation of the device 10. A real-time clock 11 is in operative connection with the calculating means 13. The real-time clock together with the calculating unit 13 is designed to create an alarm according to a predetermined measurement schedule.

The device 10 further comprises an EEPROM memory 12 which is in operative connection with the calculating means 13. Measurement results and/or a measurement schedule may be stored in the memory 12. The device 10 furthermore may be optionally provided with a speaker 19 for indicating an audible alarm, with a printer 20 or with an interface for connection to a personal computer 21.

Fig. 3 shows in some more details the display and user interface part of the device 10 according to the invention. A memory button 18 may be used for displaying average values stored in a memory as will be described hereinafter. An on/off button 22 is used to turn the device 10 on or off. A start button 23 is used to start the measurement of the blood pressure values.

The display 14 comprises a plurality of different display sections. In a systolic blood pressure display section 30, systolic blood pressure values are displayed. In a diastolic blood pressure display section 31, diastolic blood pressure values are displayed. In a pulse display section 32, pulse values may be displayed.

Furthermore, the device may comprise a medication display section 33. In this section, the user may be reminded to take his or her medication according to a predetermined schedule. A medication display section 33 is especially used in context with a therapeutic measurement mode.

In a measurement day display section 34, the number of the day in a sequence of predetermined measurement days may be indicated. Typically, during diagnostic measurements, measurements are made on seven successive days.

In an alarm display section 35, an alarm symbol may be displayed.

In an irregular heartbeat display section 36, the occurrence percentage of irregular heartbeat measurements during a predefined measurement period, in particular during the measurements which have been made according to the measurement schedule may be displayed. In an irregular heartbeat trend display section 37, the change, in particular increase or decrease of the occurrence percentage of irregular heartbeats may be displayed.

In a time and date display section 38, the date and the current time may be indicated.

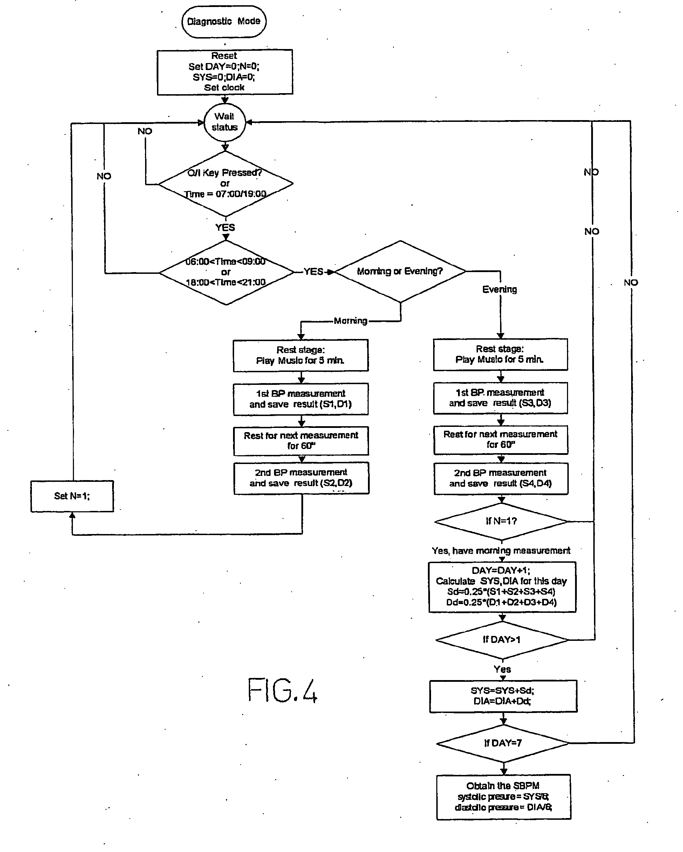

Fig. 4 shows a flow chart for operation of the device of the present invention in a first, so-called diagnostic mode. For this diagnostic mode, blood pressure measurements shall be made for a predetermined number of days, in particular seven days for one specific patient. At the start of this measurement sequence, parameter values in the device 10 are set to zero. In particular, temporary variables DIA and SYS for the systolic and diastolic blood pressure, the number of measurement days and a parameter N relating to measurements within the day are set to zero.

The device 10 is then put into a waiting mode. It is waited until the on/off key 22 shown in Fig. 3 is pressed or until the time is 7 am or 7 pm. If one of these conditions is fulfilled, in a next step, it is verified whether the current time is between 6 or 9 am or between 6 and 9 pm. If this is not the case, the device is put back in a waiting condition. By this step, it is made sure that the user cannot make measurements at inappropriate time intervals, e. g. at times lying outside the 6 to 9 am/pm range. When the user presses the start button 23 (Fig. 3), the measurement procedure will start.

If the time is within this range, it is determined whether the time is before or after noon. If the time is in the morning, a morning measurement procedure is started. If the time is in the evening, a different evening measurement procedure is started.

In the morning measurement procedure, in a first stage, the user is asked to rest for five minutes. Optionally, the device may play music during this time. Playing of music can be disabled/enabled by the user or by a doctor. After this rest period which allows stabilising the blood pressure, a first blood pressure measurement is made. The results S1, D1 for the systolic and diastolic blood pressure are saved in the memory 12.

There follows a rest period before taking another measurement. The rest period typically may be 60 seconds. After this rest period, a second blood pressure measurement is done. The results for the systolic blood pressure S2 and the diastolic blood pressure D2 are saved. After completion of this morning measurement procedure, the parameter value N is set to 1 indicating that a morning measurement has been made.

The measurement procedure for the evening measurement is substantially identical to the morning measurement. Two blood pressure measurements are made whereby systolic blood pressure values S3, S4 and diastolic blood pressure values D3, D4 are determined and saved. After termination of this procedure, it is verified whether the parameter N is equal to 1. If no, it is judged that there was no morning measurement of the same day and the device is put back into the waiting status.

If there was a morning measurement, the parameter for the measurement day is increased by one. A systolic and diastolic average blood pressure value Sd, Dd for this day is calculated by summing the stored values D1, D2, D3, D4 and S1 S2, S3, S4 respectively and by dividing them by 4.

If these are measurements of the first measurement day, i. e. if the parameter DAY is equal to 1, the parameter values are disregarded and the device is set back into the waiting status.

If the measurements were not made on the first measurement day, operation is continued and the calculated daily averages Sd, Dd are added to variables SYS and DIA which are temporary variables for the systolic and diastolic blood pressure.

If the measurement day is not equal to 7, the device is set back intro the waiting status. If the measurement day is equal to 7, blood pressure measurements in the morning and in the evening of seven days have been successfully made. Consequently, an average blood pressure value for the systolic pressure and for the diastolic pressure is obtained by dividing the content of the temporary variables SYS and DIA by 6. Division by six is made because measurements of the first day are disregarded.

By this method, it is made sure that measurements of six days are considered. Measurements of the first day are disregarded. Only days where measurements in the morning and in the evening have been made are considered. All measurements have been made in the predetermined time window.

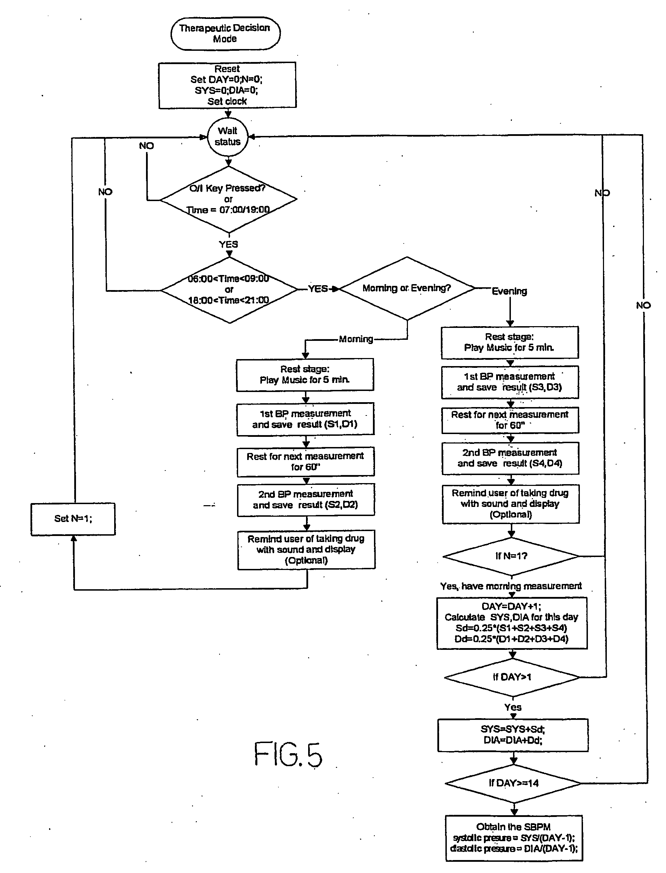

Fig. 5 shows a flow chart of the device operating in the therapeutic operation mode. Operation is substantially identical to the one as shown in Fig. 4 with the following differences: After completion of the morning and evening measurement procedure, the user is reminded to take a drug. Indication can be made by sound and/or by a display.

Contrary to the flow chart shown in Fig. 4, daily measurements are continued for fourteen days, i. e. until the variable DAY is equal or above 14.

If this condition has been reached, average systolic pressure values and diastolic pressure values are calculated by dividing the variables SYS and DIA through a number corresponding to the number of days minus 1. One day is subtracted because measurement values of the first day are disregarded during formation of averages.

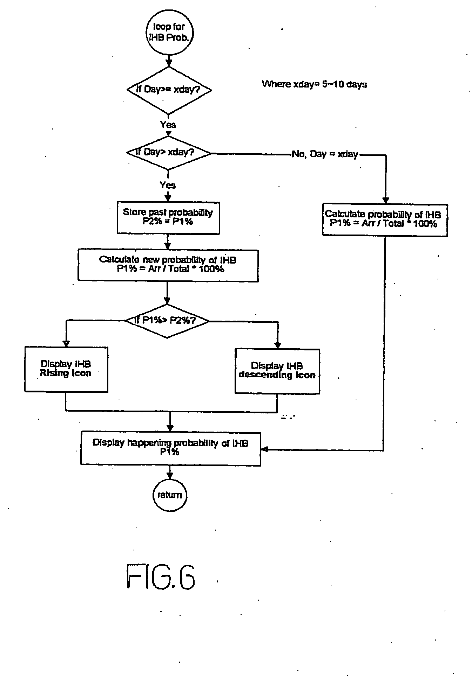

Fig. 6 shows a flow chart of an irregular heartbeat probability detection mode. In a first step, it is determined how many measurements on successive days have been made. If there are less than a predetermined number of days, in particular less than 5 to 10 subsequent measurement days, an irregular heartbeat probability is calculated and displayed.

If there have been more measurement days than a predetermined number xday of days, the past occurrence percentage of irregular heartbeats P1% is stored in a variable P2 %. A new percentage P1% is calculated. Depending on the relationship between these probabilities P1% and P2%, an increase or a decrease of occurrence percentage of irregular heartbeat measurements is displayed by means of a rising icon or a descending icon. In addition, the percentage of irregular heartbeats also will be displayed. Determination of the percentage or number or irregular heartbeats may be made in a known manner.

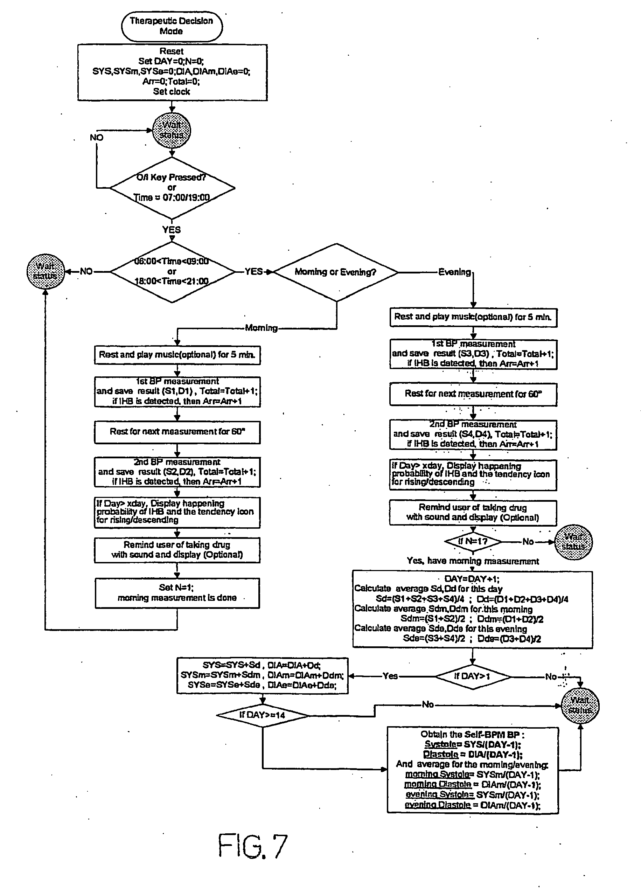

In Fig. 7, a flow chart for an alternative therapeutic decision mode is shown. In addition to the flow chart of Fig. 5, there is irregular heartbeat detection. For irregular heartbeat or arrhythmia, a variable "Arr" is used. At the beginning of the procedure the variable "Arr" and also a variable "total" relating to the number of measurements will be set to zero. In addition, average values for evening and morning measurements for the systolic and diastolic blood pressure are used and will also be set to zero.

In addition to the procedure explained with, reference to Fig. 5, during each measurement, the variable total will be increased by 1. Furthermore, if a irregular heartbeat is detected during one measurement, a variable "Arr" will be increased by 1.

If the variable "day" indicating the number of measurement days is above a predetermined value xday (see also Fig. 6) the percentage of irregular heartbeats and a rising/descending tendency of irregular heartbeats will be displayed.

In addition to the procedure shown with reference to Fig. 5, in the procedure shown in Fig. 7 morning and evening averages are also calculated for the systolic and diastolic blood pressure.

## Claims

1. A blood pressure measuring device (10) for self-monitoring the blood pressure of a patient,
wherein said device (10) comprises an alarm generator (11) designed in such a way as to remind the patient to carry out a blood pressure measurement according to a predetermined measurement schedule
or to automatically switch on the device according to a predetermined measurement schedule,
**characterised in that** said device is designed in such a way that measurements can only be made in a certain time window, around an alarm time, said device further comprising means for blocking operation of said device outside said time window.

2. A blood pressure measuring device according to claim 1,
wherein said device (10) is provided with a memory (12) having said measurement schedule stored therein.

3. A blood pressure measuring device according to one of the claims 1 or 2, wherein the device (10) comprises a memory (12) for storing results of measurements made according to said measurement schedule.

4. A blood pressure measuring device according to claim 3,
wherein the device (10) comprises a calculating means (13) for forming averages of the results of at least two measurements, in particular for forming daily averages based on at least two measurements made on one day and/or for forming averages of a plurality of daily measurements.

5. A blood pressure measuring device according to claim 4, said device (10) further comprising means (14, 18) for displaying said averages.

6. A blood pressure measuring device according to one of the claims 4 or 5, wherein said calculating means (13) for forming averages are designed in such a way as to disregard measurements made on the first day of a series of subsequent daily measurements.

7. A blood pressure measuring device according to one of the claims 1 to 6, wherein said measurement schedule generates a daily morning and a daily evening alarm which is repeated for a predetermined number of days, preferably for at least three, in particular for seven subsequent days.

8. A blood pressure measuring device according to one of the claims 1 to 7, wherein said device (10) is operatable in a diagnostic mode based on said measurement schedule and in at least one of a regular mode and a therapeutic mode.

9. A blood pressure measuring device according to claim 8, wherein said device is provided which a switch (15) for manually switching between said operating modes.

10. A blood pressure measuring device (10), according to one of the claims 1 to 9, comprising
- means for detecting irregular heartbeats
- means for storing occurrence of an irregular heartbeat
- means for calculating an occurrence percentage of irregular heartbeat measurements within a predetermined period of time and
- means (14, 36) for displaying said occurrence percentage.

11. A blood pressure measuring device according to claim 10,
wherein the device (10) comprises calculating means for determining an increase or decrease of said occurrence percentage and means (37) displaying said increase or decrease.

12. A method for operating a blood pressure measuring device (10), in particular a device according to one of the claims 1 to 11, comprising the steps of
- providing a predetermined measurement schedule
- when a criteria of said schedule is fulfilled, generating an alarm signal reminding a patient to carry out a measurement or switching on said device (10),
**characterised in that** the method comprising the further step of blocking operation of the device (10) if the current time is not within a predetermined time window around an alarm time.

13. A method according to claim 12 , wherein an alarm is generated daily in the morning and in the evening for a plurality of successive days, in particular for seven successive days.

14. A method according to one of the claims 12 or 13 wherein averages of a plurality of measurement values are formed, in particular wherein daily averages and averages of a plurality of subsequent daily measurements are formed.

15. A method according to claim 14, wherein measurements of the first measurement day are disregarded for calculation of said averages.

16. A method according to one of the claims 12 to 15, comprising the steps of
- detecting irregular heartbeats
- calculating an occurrence percentage of irregular heartbeat measurements in a predetermined period of time
- displaying said occurrence percentage.

17. A method according to claim 16, comprising the further step of detecting an increase or decrease of said occurrence percentage.

## Patentansprüche

1. Blutdruckmessgerät (10) zur Selbstüberwachung des Blutdrucks eines Patienten, worin das Gerät (10) einen Alarmgenerator (11) umfasst, so dass der Patient daran erinnert wird, eine Blutdruckmessung nach einem vorbestimmten Messzeitplan durchzuführen, oder so dass das Gerät nach einem vorbestimmten Messzeitplan automatisch angeschaltet wird, **dadurch gekennzeichnet, dass** das Gerät so konzipiert ist, dass Messungen nur innerhalb eines bestimmte Zeitfensters durchgeführt werden können, wobei das Gerät ferner ein Mittel zum Blockieren des Betriebs des Geräts außerhalb des Zeitfensters um eine Alarmzeit umfasst.

2. Blutdruckmessgerät nach Anspruch 1, wobei das Gerät (10) mit einem Speicher (12), in dem der Messzeitplan gespeichert ist, ausgestattet ist.

3. Blutdruckmessgerät nach einem der Ansprüche 1 oder 2, wobei das Gerät (10) einen Speicher (12) zum Speichern der Ergebnisse der Messungen, die nach dem Messzeitplan durchgeführt wurden, umfasst.

4. Blutdruckmessgerät nach Anspruch 3, wobei das Gerät (10) ein Rechenmittel (13) zum Bilden von Durchschnittswerten der Ergebnisse von mindestens zwei Messungen, insbesondere zum Bilden von täglichen Durchschnittswerten auf Basis von mindestens zwei Messungen an einem Tag und/oder zum Bilden von Durchschnittswerten einer Vielzahl von täglichen Messungen umfasst.

5. Blutdruckmessgerät nach Anspruch 4, wobei das Gerät (10) ferner Mittel (14, 18) zum Anzeigen der Durchschnittswerte umfasst.

6. Blutdruckmessgerät nach einem der Ansprüche 4 oder 5, wobei das Rechenmittel (13) zum Bilden von Durchschnittswerten so konzipiert ist, dass es Messungen am ersten Tag einer Reihe von aufeinanderfolgenden Messtagen außer Acht lässt.

7. Blutdruckmessgerät nach einem der Ansprüche 1 bis 6, wobei der Messzeitplan einen täglichen morgendlichen und einen täglichen abendlichen Alarm erzeugt, der über eine vorbestimmte Anzahl Tage, vorzugsweise mindestens drei Tage, insbesondere sieben aufeinanderfolgende Tage, wiederholt wird.

8. Blutdruckmessgerät nach einem der Ansprüche 1 bis 7, wobei das Gerät (10) in einem Diagnosemodus auf Basis des Messzeitplans und in mindestens einem regulären Modus und einem Therapiemodus bedient werden kann.

9. Blutdruckmessgerät nach Anspruch 8, wobei das Gerät mit einem Schalter (15) zum manuellen Umschalten zwischen den Betriebsmodi ausgestattet ist.

10. Blutdruckmessgerät (10) nach einem der Ansprüche 1 bis 9, die Folgendes umfasst:
- ein Mittel zum Nachweis von unregelmäßigem Herzschlag
- ein Mittel zum Speichern des Auftretens von unregelmäßigem Herzschlag
- ein Mittel zum Berechnen des prozentualen Auftretens von unregelmäßigem Herzschlag innerhalb eines vorbestimmten Zeitraums und
- Mittel (14, 36) zum Anzeigen des prozentualen Auftretens.

11. Blutdruckmessgerät nach Anspruch 10, wobei das Gerät (10) ein Rechenmittel zum Bestimmen einer Zunahme oder Abnahme des prozentualen Auftretens und ein Mittel (37) zum Anzeigen der Zu- bzw. Abnahme umfasst.

12. Verfahren zum Bedienen eines Blutdruckmessgeräts (10), insbesondere eines Geräts nach einem der Ansprüche 1 bis 11, das folgende Schritte umfasst:
- Bereitstellen eines vorbestimmten Messzeitplans
- wenn ein Kriterium des Zeitplans erfüllt ist Erzeugen eines Alarmsignals, um den Patienten daran zu erinnern, eine Messung durchzuführen, oder um das Gerät (10) anzuschalten, **dadurch gekennzeichnet, dass** das Verfahren den weiteren Schritt der Blockierung des Betriebs des Geräts (10) umfasst, wenn die aktuelle Zeit nicht innerhalb eines vorbestimmten Zeitfensters um einen Alarmzeitpunkt liegt.

13. Verfahren nach Anspruch 12, wobei täglich morgens und abends über eine Vielzahl von aufeinanderfolgenden Tagen, insbesondere sieben aufeinanderfolgende Tage, ein Alarm erzeugt wird.

14. Verfahren nach einem der Ansprüche 12 oder 13, wobei Durchschnittswerte einer Vielzahl von Messwerten gebildet werden, insbesondere worin tägliche Durchschnittswerte und Durchschnittswerte einer Vielzahl von aufeinanderfolgenden täglichen Messungen gebildet werden.

15. Verfahren nach Anspruch 14, wobei Messungen des ersten Messtages bei der Berechnung der Durchschnittswerte außer Acht gelassen werden.

16. Verfahren nach einem der Ansprüche 12 bis 15, das die folgenden Schritte umfasst:
- Nachweis von unregelmäßigem Herzschlag
- Berechnen eines prozentualen Auftretens von unregelmäßigem Herzschlag innerhalb eines vorbestimmten Zeitraums
- Anzeige des prozentualen Auftretens.

17. Verfahren nach Anspruch 16, das den weiteren Schritt des Nachweises einer Zu- oder Abnahme des prozentualen Auftretens umfasst.

## Revendications

1. Dispositif (10) de mesure de la pression artérielle permettant à un patient de surveiller lui-même sa pression artérielle, ledit dispositif (10) comprenant un générateur d'alarme (11) conçu pour générer une alarme de manière à rappeler au patient d'effectuer une mesure de pression artérielle conformément à un horaire de mesures prédéfini ou pour mettre automatiquement le dispositif sous tension conformément à un horaire de mesures prédéfini,
**caractérisé en ce qu'**il est conçu de manière à ne permettre les mesures qu'au sein d'une certaine fenêtre horaire,
ledit dispositif comprenant en outre des moyens destinés à bloquer le fonctionnement dudit dispositif en dehors de ladite fenêtre horaire contenant une heure d'alarme.

2. Dispositif de mesure de la pression artérielle selon la revendication 1, ledit dispositif (10) étant pourvu d'une mémoire (12) dans laquelle est mémorisé ledit horaire de mesures.

3. Dispositif de mesure de la pression artérielle selon l'une des revendications 1 ou 2, le dispositif (10) comprenant une mémoire (12) destinée à mémoriser des résultats de mesures effectuées conformément audit horaire de mesures.

4. Dispositif de mesure de la pression artérielle selon la revendication 3, le dispositif (10) comprenant un moyen de calcul (13) destiné à former des moyennes des résultats d'au moins deux mesures, plus particulièrement à former des moyennes quotidiennes à partir d'au moins deux mesures effectuées sur un jour et/ou à former des moyennes d'une pluralité de mesures quotidiennes.

5. Dispositif de mesure de la pression artérielle selon la revendication 4, ledit dispositif (10) comprenant en outre des moyens (14, 18) destinés à afficher lesdites moyennes.

6. Dispositif de mesure de la pression artérielle selon l'une des revendications 4 ou 5, ledit moyen de calcul (13) destiné à former des moyennes étant conçu de manière à ne pas tenir compte des mesures effectuées le premier jour d'une série de mesures quotidiennes consécutives.

7. Dispositif de mesure de la pression artérielle selon l'une des revendications 1 à 6, ledit horaire de mesures générant une alarme quotidienne le matin et une alarme quotidienne le soir répétées pendant un nombre prédéfini de jours, de préférence au moins trois, plus particulièrement pendant sept jours consécutifs.

8. Dispositif de mesure de la pression artérielle selon l'une des revendications 1 à 7, ledit dispositif (10) pouvant fonctionner dans un mode diagnostic en fonction dudit horaire de mesures et dans un mode normal et/ou un mode thérapeutique.

9. Dispositif de mesure de la pression artérielle selon la revendication 8, ledit dispositif étant pourvu d'un commutateur (15) destiné à permettre une commutation manuelle entre lesdits modes de fonctionnement

10. Dispositif (10) de mesure de la pression artérielle selon l'une des revendications 1 à 9, comprenant :
- des moyens destinés à détecter un rythme cardiaque irrégulier,
- des moyens destinés à mémoriser l'apparition d'un rythme cardiaque irrégulier,
- des moyens destinés à calculer un pourcentage d'apparition de mesures de rythme cardiaque irrégulier au sein d'un intervalle de temps prédéfini, et
- des moyens (14, 36) destinés à afficher ledit pourcentage d'apparition.

11. Dispositif de mesure de la pression artérielle selon la revendication 10, le dispositif (10) comprenant des moyens de calcul destinés à déterminer une augmentation ou une diminution dudit pourcentage d'apparition et un moyen (37) destiné à afficher ladite augmentation ou ladite diminution.

12. Procédé de fonctionnement d'un dispositif (10) de mesure de la pression artérielle, plus particulièrement d'un dispositif selon l'une des revendications 1 à 11, comprenant les étapes consistant à :
- établir un horaire de mesures prédéfini,
- si un critère dudit horaire est satisfait, générer un signal d' alarme de manière à rappeler à un patient d'effectuer une mesure ou à mettre ledit dispositif (10) sous tension,
**caractérisé en ce qu'**il comprend en outre l'étape consistant à bloquer le fonctionnement du dispositif (10) si l'heure actuelle sort d'une fenêtre horaire prédéfinie contenant une heure d'alarme.

13. Procédé selon la revendication 12, une alarme étant générée quotidiennement le matin et le soir pendant une pluralité de jours consécutifs, plus particulièrement pendant sept jours consécutifs.

14. Procédé selon l'une des revendications 12 ou 13, des moyennes d'une pluralité de valeurs de mesure étant formées, plus particulièrement des moyennes quotidiennes et des moyennes d'une pluralité de mesures quotidiennes consécutives étant formées.

15. Procédé selon la revendication 14, les mesures du premier jour de mesure n'étant pas prises en compte dans le calcul desdites moyennes.

16. Procédé selon l'une des revendications 12 à 15, comprenant les étapes consistant à :
- détecter un rythme cardiaque irrégulier,
- calculer un pourcentage d'apparition de mesures de rythme cardiaque irrégulier au sein d'un intervalle de temps prédéfini, et
- afficher ledit pourcentage d'apparition.

17. Procédé selon la revendication 16, comprenant en outre l'étape consistant à détecter une augmentation ou une diminution dudit pourcentage d'apparition,
